# EUROPEAN PATENT APPLICATION

(11) **EP 4 310 852 A1**
(43) Date of publication of application: **24.01.2024**
(21) Application number: 22185496.1
(22) Date of filing: 18.07.2022
(51) Int. Cl.: G16H 30/00, G16H 30/20, G16H 30/40

(54) **SYSTEMS AND METHODS FOR MODIFYING IMAGE DATA OF A MEDICAL IMAGE DATA SET**

(71) Applicant: Siemens Healthcare GmbH, 91052 Erlangen (DE)
(72) Inventor: Hölzer, Philipp, 91088 Bubenreuth (DE); Laugerette, Alexis, 91058 Erlangen (DE)
(74) Representative: Siemens Healthineers Patent Attorneys

(57) **Abstract**

Methods and systems are provided for enhancing a medical image data set for making corresponding image analysis results better accessible for a user. In particular, it is suggested to obtain the medical image data set, the medical image data set depicting an image volume and comprising a plurality of slices with image data depicting a certain section of the image volume, to obtain at least one image analysis result applicable to the medical image data set, to determine a positional information of the image analysis result in the medical image data set, to modify the image data of one or more slices of the medical image data based on the positional information so that the modified image data highlights the position of the image analysis results in the medical image data set, and to provide the modified image data.

## Description

The invention relates to systems and methods for modifying image data comprised in a medical image data set. In particular, the invention relates to systems and methods for modifying the image data such that image analysis results related to the medical image data set are highlighted in the medical image data set.

The emergence of AI-powered applications in medical imaging offers a user, such as a radiologist or other physician, a whole new toolset for the automatic analysis of medical image data. In the medical field, such tools are often referred to as computer-aided detection (CADe) or computer-aided diagnosis (CADx) algorithms. Hereafter both types of systems will be referred to as CAD algorithms. CAD algorithms are technologies to help radiologists interpret medical images. A common use of CAD algorithms is to automatically identify suspicious regions in a medical image. Such suspicious regions may contain image patterns indicative of abnormalities which may comprise cancerous growths, masses, abscesses, lacerations, calcifications, lesions and/or other irregularities within biological tissue.

Basically, an ideal CAD algorithm should be able to securely identify all actual abnormalities without generating any false positives. This may sound straightforward but is very difficult to achieve in practice. By consequence, there exists a plethora of different highly specialized algorithms directed to specific organs and/or clinical findings. As a consequence, such CAD algorithms often come from specialized vendors which may be different from the vendor providing the diagnostic workplace at which the user is reviewing the medical image data set.

By consequence, an issue arises when these results are to be integrated in the diagnostic workflow running on the diagnostic workplace. This is even more so as a common standard for the interoperability of suchlike image analysis results among various vendors is still at large. By consequence, most CAD algorithms available today in the field of radiology are outputting DICOM secondary captured images, which represent burnt-in annotations or measurements made automatically by the CAD algorithms.

Navigating these image analysis results is a difficult task for the user, since these results can be tiny datapoints in a large dataset (for example, one single distance measurement in one single slice within a large CT dataset of 1000+ slices). This task gets even more complex when the number of different CAD algorithms or the number of resulting secondary capture series scales. Moreover, there is a certain risk that individual image analysis results are overlooked which can cause serious medical problems.

It is an object of the present invention to provide methods and systems which help the user to better access image analysis results related to the medical image data. In particular, it is an object of the present invention to facilitate the access to image analysis results associated with the medical image data set and to minimize the cognitive effort associated with this kind of navigation.

This object is solved by a method for modifying image data of a medical image data set, a corresponding system, a corresponding computer-program product, and a computer-readable storage medium according to the independent claims. Alternative and/or preferred embodiments are object of the dependent claims.

In the following, the technical solution according to the present invention is described with respect to the claimed apparatuses as well as with respect to the claimed methods. Features, advantages, or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

According to an aspect, a computer-implemented method for modifying image data of a medical image data set is provided. The method has a plurality of steps. One step is directed to obtaining the medical image data set, the medical image data set depicting an image volume and comprising a plurality of slices with image data depicting a certain section of the image volume. Another step is directed to obtaining at least one image analysis result applicable to the medical image data set. Another step is directed to determining a position of the image analysis result in the medical image data set. Another step is directed to modifying the image data of one or more slices of the medical image data set so that the modified image data indicates (or highlights) the position of the image analysis results in the medical image data set. Another step is directed to providing the modified image data.

The medical image data set may relate to a medical image study. Such medical image studies may relate to three-dimensional data sets providing three dimensions in space. The medical image data set may depict a body part of a patient in the sense that it contains three-dimensional image data of the patient's body part. The medical image data set is representative of an image volume. The patient's body part may be comprised in the image volume.

The medical image data set comprises image data, for example, in the form of a three-dimensional array of pixels or voxels. Such arrays of pixels or voxels may be representative of intensity, absorption or other parameters as a function of three-dimensional position, and may, for example, be obtained by suitable processing of measurement signals obtained by a medical imaging modality.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a specially rotating instrument. The resulting attenuation data (also referred to as raw data) is presented by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

Accordingly, the depicted body part of the patient in general will comprise a plurality of anatomies and/or organs. Taking a chest image as an example, the medical image data set may show lung tissue, the rib cage, lymph nodes and others.

The medical image data set comprises a plurality of images or image slices. The slices respectively show a cross-sectional view of the image volume. The slices may comprise a two-dimensional array of pixels or voxels as image data. The arrangement of slices in the medical image data set may be determined by the imaging modality or by any post-processing scheme used. Further, slices may artificially be defined in the imaging volume spanned by the medical image data set. Optionally, this may happen as a function of the image data comprised in the medical image data set in order to optimally pre-process the medical image data set for the ensuing diagnostic workflow.

The medical image data set may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

"Obtaining" may mean that the medical image data set is directly obtained from the medical imaging modalities. Further "obtaining" may mean that the medical image data set is retrieved from an appropriate memory such as a picture archiving and communication system (PACS) or any other suitable medical image storing facility.

The Image analysis result may comprise the results of an analysis process of image data related to the medical image data set. Thereby, the image data related to the medical imaged data may be the image data of the medical image data set itself or image data of a related medical image data set. Such related medical image data set may, for instance, have been acquired from the same patient as the patient of the medical image data set but at a different point in time. Further, a related medical image data set may be acquired from a different (but, preferably, similar) patient than the patient of the medical image data set.

The image analysis process may be a manual process carried out by a user. Further, the image analysis process may involve inputting image data into an image analysis algorithm configured to output an image analysis result.

The image analysis result may comprise one or more individual medical findings. Each medical finding may pertain to corresponding image data in the medical image data set. A medical finding may indicate a certain condition or pathology of the patient which is relevant for the diagnosis of the patient. A medical finding may be an anatomical structure that differentiates the patient from other patients. A medical finding may be located within different organs of the patient (e.g., within the lung of a patient, or within the liver of a patient) or in between the organs of the patient. In particular, a medical finding may be a foreign body.

In particular, a medical finding may be a neoplasm (also denoted as "tumor"), in particular, a benign neoplasm, an in situ neoplasm, an malignant neoplasms and/or a neoplasms of uncertain/unknown behavior. In particular, a medical finding can be a nodule, in particular, a lung nodule. In particular, a medical finding may be a lesion, in particular, a lung lesion.

The positional information of the image analysis result may relate to the position within the medical image data set, where the image analysis result refers to. For instance, the positional information may provide the position of image data within the medical image data set which relates to the image analysis result. In particular, the positional information may indicate one or more slices corresponding to the image analysis result. Further, the positional information may indicate coordinates corresponding to the image analysis result within the image volume or within individual slices representing the image volume.

The positional information may comprise the position of each medical finding within the medical image data set. For instance, the positional information may provide the position of image data within the medical image data set which represents the one or more medical findings. In particular, the positional information may provide, for each medical finding, the slice position, i.e., indicate the slice where the medical finding is to be found. Further, the positional information may provide, for each medical finding, the coordinates within the respective slice or the image volume where the medical finding resides / was identified.

In particular, the step of modifying may comprise modifying the image data such that it indicates the position of the image analysis may comprise including a highlighting, visual directory, or other marking in the image data that helps the user finding the image analysis result. Thereby, also image data different from the image data directly corresponding to the image analysis results may be modified. For instance, if the image analysis results correspond to a number of distinct slices of the medical image data set, the step of modifying may comprise modifying the image data of the distinct slices and of the other slices (i.e., other than the distinct slices) of the medical image data set.

Determining the positional information of the image analysis results enables to enrich or enhance (or, in general, process) the medical image data set so as to show additional optical clues for the user directed to the image analysis results. This is provided for by specifically modified image data indicating the position of the image analysis result in the medical image data set for the user. Upon scrolling through the medical image data set, the user is guided to the image analysis results. In general, the positional information may be seen as relating to an internal state of the medical image data set. By including this in the image data, the user is enabled to properly operate a diagnosis system when reviewing the medical image data. Accordingly, a user can more easily and efficiently review the medical image data set for contents which are potentially relevant for her or his diagnosis. At the same time, there is a reduced risk of missing important parts of the medical image data set.

According to an aspect, the step of providing the modified image data comprises generating a processed medical image data set based on the image data set and the modified image data and providing the processed medical image data set.

In particular, the modified image data may be included in the processed medical image data set. In particular, the modified image data may replace the original image data it was modified from in the processed medical image data set.

In other words, the modified image data is "burnt-in" in the processed medical image data set. With that, the image processing result of the present invention resulting in modified image data indicating the position of the image analysis results is better accessible and portable between different workstations and applications.

According to an aspect, the processed medical image data set comprises a DICOM secondary capture image information object.

Thereby, each slice of the processed medical image data set may be a DICOM secondary capture image object. Further, only slices with modified image data may be DICOM secondary capture image objects.

DICOM secondary capture image objects are a class of image objects defined in the DICOM standard. DICOM secondary capture images may be seen as the "lowest common denominator" for images available in DICOM. Whilst there is sufficient information to identify the patient, date, and institution, they do not contain any scaling, or other modality information, so are effectively "just pictures". Often, the Secondary Capture (SC) Image Information Object Definition (IOD) specifies images that are converted from a non-DICOM format to a modality independent DICOM format. DICOM SC images are created, for example, by image analysis algorithms analyzing image data and "inscribing" the analysis results in the image data. As such, DICOM images can hold important curated clinical data that allow a better understanding of the patient case.

Using the DICOM secondary caption class for the processed medical image data set makes it vendor and modality independent. This has the benefit that it becomes more portable and more readily usable in the diagnostic workflow.

According to an aspect, the step of providing the modified image data comprises generating a representation for display to a user on the basis of the modified image data and displaying the representation.

According to this aspect, the modified image data is directly used for visualizing the medical image data set. With that, a user can immediately use and interact with the information encoded in the modified image data.

According to an aspect, the at least one image analysis result is included in the medical image data set in the form of one or more annotations on one or more slices of the medical image data set.

In particular, the one or more annotations are included in the image data of the medical image data set.

In particular, the one or more annotations may be represented by image data that has been modified from original image data of the medical image data set so as to show the one or more annotations. In particular, the image data representing the one or more annotations may have been modified from original image data upon generation of the image analysis results. In particular, the one or more annotations may respectively represent one or more medical findings.

In particular, the medical image data set comprises one or more DICOM secondary capture image information objects.

An annotation may identify a region in the medical image data set. Specifically, the annotation may comprise a (visual) outline delineating the region. For instance, the outline may be of circular, rounded, rectangular, or irregular shape. Further, an annotation may comprise text and/or numbers. Further, an annotation may comprise measurement values. The annotation may be generated manually by a user upon reviewing an original medical image data set underlying the medical image data set. Alternatively, the annotations may be generated by one or more image analysis algorithms.

Including the image analysis results in the medical image data set (or in the image data of the medical image data set) may allow for a swift exchange of image analysis results. Further, an improved interoperability between different vendors and image analysis applications can be ensured. As discussed, this may come at the cost of the image analysis results being embedded in the medical image data and not being very well accessible for a user. This drawback is alleviated, however, with the additional modification of image data according to the present invention so as to indicate the position of the image analysis results.

According to an aspect, the one or more annotations are generated by processing an original medical image data set with one or more image analysis algorithms, the original image data set representing the medical image data set before processing with the one or more image analysis algorithms.

In other words, the medical image data set comprising one or more annotations may be seen as the output of one or more image analysis algorithms applied to original image data.

According to an aspect, the positional information is provided separately by the one or more image analysis algorithms. In other words, the positional information is provided as supplementary information together with the image analysis results. This has the advantage that the positional information is immediately usable for the further processing.

According to an aspect, the positional information is included in metadata associated with the medical image data set and the step of determining the positional information comprises analyzing the metadata for the positional information.

For instance, the positional information may be comprised in a header of the medical image data set. For instance, in the case of the medical image data set being formatted according to the DICOM standard, the positional information may be stored in one or more DICOM attributes.

According to an aspect, the step of determining the positional information comprises: providing an image processing algorithm configured to recognize annotations in a medical image data set and provide positional information of the recognized annotations, and inputting the medical image data set (comprising the one or more annotations) in the image processing algorithm so as to determine the positional information.

The positional information may comprise the position of each annotation within the medical image data set. For instance, the positional information may provide the position of image data within the medical image data set specified by the one or more annotations. In particular, the positional information may provide, for each annotation, the slice position, i.e., indicate the slice where the annotation is to be found. Further, the positional information may provide, for each annotation, the coordinates within the respective slice or the image volume where the annotation resides.

In particular, the image processing algorithm may be configured to recognize non-physiological data in a medical image data set based on an analysis of pixel values.

Due to their uniform brightness or colour values and comparably uniform shapes, the annotations may be relatively easily recognized as non-physiological data. Once recognized, it is straight-forwardly possible to also get the positional information of the annotations.

If, in addition to that, also the meaning or one or more characteristics of the annotations is to be extracted, the image processing algorithm may also be implemented as character or object recognition algorithm. In particular, optical character recognition OCR may be used to extract one or more characters associated with medical findings or annotations from the medical image data set. OCR is the electronic conversion of images of typed, handwritten or printed text into machine-encoded text. Various OCR software is available and may be applied to this disclosure, such as, Tesseract Open Source OCR as presented in non-patent literature - Kay, Anthony. "Tesseract: an open-source optical character recognition engine." Linux Journal 2007.159 (2007):2.

According to an aspect, the step of modifying the image data comprises modifying the image data such that the modified image data indicates the position of the image analysis results in addition to the one or more annotations.

In particular, the image data of the annotations may remain unchanged in the modifying step.

Summarizing some of the above explanations, according to a further aspect, a method for modifying image data of a medical image data set may be provided which has a plurality of steps. On step is directed to obtaining the medical image data set, wherein the medical image data set depicts an image volume and comprises a plurality of slices with image data depicting a certain section of the image volume, and wherein the medical image data set comprises one or more annotations on one or more of the slices, the one or more annotations relate to at least one image analysis result applicable to the medical image data set. Another step is directed to determining positional information of the image analysis result (or the annotations) in the medical image data set, preferably based on the medical image data set, more preferably by applying an image processing algorithm to the medical image data set configured to recognize annotations in a medical image data set and provide positional information of the recognized annotations. Another step is directed to modifying the image data of one or more slices of the medical image data set based on the positional information so that the modified image data indicates the position of the image analysis result (or the annotations) in the medical image data set. Another step is directed to providing the modified image data.

According to an aspect, the image data of the plurality of slices respectively comprises a plurality of pixels each having a pixel value, and the step of modifying comprises changing one or more pixel values of the one or more slices (based on the positional information).

In other words, the modifications based on the positional information are "burnt-in" in the medical image data set. With that, the newly included information is made portable and can also more readily be saved for later use. With that, a user can more easily and efficiently come back to the findings comprised in the medical image data set and, thus, more easily and efficiently review the medical image data set.

According to an aspect, in the step of modifying, the image data of all slices comprised in the medical image data set is modified (based on the positional information).

This has the advantage that guidance for a user may be provided in all the slices of the medical image data set. Accordingly, the user gets an indication concerning the position of image analysis results in the medical image data set no matter where she or he starts with the inspection of the data set. With that, a user can more easily and efficiently review the medical image data set for contents which are potentially relevant for her or his diagnosis.

According to an aspect, the step of modifying comprises modifying the image data of the slices of the medical image data set so that, for each slice, the modified image data indicates (or depicts) the position of the respective slice in the medical image data set and, relative thereto, the slice position(s) of the image analysis result.

In other words, two pieces of information are imprinted in the slices: the current position and positions of potentially interesting slices. It thus becomes derivable from each slice where the slice resides in the medical image data set and where the "interesting" slices are which comprise one or more findings / annotations. With that, a user can infer where in the medical image data set she or he currently is and where she or he needs to go for the interesting parts. Without that information, the user would have no indication in which direction to move. Instead, she or he would have to go through the medical image data set in a more erratic manner. Accordingly, the user is brought into a position to more easily and efficiently review the medical image data set for contents which are potentially relevant for her or his diagnosis.

If a diagnostic workstation is considered, where the user has to review medical image data sets, the annotations comprised in different medical image data sets would relate to the internal state of the workstation, i.e., the data loaded in the workstation. Thereby, the location of the annotations is not static but changes from medical image data set to medical image data set. Systematically including this information in the data the user needs to review, the user is provided with an indication of that internal state which brings her or him into a position to proper operate the system.

According to an aspect, the step of modifying comprises modifying the image data of the slices such that each slice depicts a virtual scrollbar representing the slices of the medical image data set, wherein the position of the respective current slice and positions of the image analysis result are respectively indicated in the scrollbar.

For instance, the positions may be indicated by visual labels in the scrollbar.

The scrollbar indicates to the user the location of the next interesting slices by dynamically displaying the current position and a guide mark regarding further interesting slices in the medical image data set. With that, a continued human-machine-interaction is facilitated by resolving conflicting technical requirements: displaying the current slice in the display area of a screen and maintaining an overview of the regions of interest within the medical image data set which cannot be displayed in the display area.

According to an aspect, the step of modifying comprises inserting one or more visual markers in the image data of the medical image data set.

According to an aspect, the visual markers comprise a color that is different from the colors otherwise comprised in the medical image data set.

Typically, the medical image data sets are provided as greyscale images. Thus, visual markers in yellow, orange, green, red or the like may serve to better notice the visual markers in the medical image data set.

According to an aspect, the visual markers are selected from: a visual indication of a region of interest, a text field, a colored margin of the respective slice, an icon, a pictogram, a measurement, a scrollbar, and/or combinations thereof.

For instance, the indication of a region of interest may be a box, rectangle, circle, or any other form.

By using visual markers, the user can more easily grasp which parts of the medical image data set are important. For instance, the user may quickly scroll to those slices of the medical image data set which comprise colored margins or are otherwise highlighted. With that, a user can more easily and efficiently review the medical image data set for contents which are potentially relevant for making a medical diagnosis.

According to an aspect, at least one visual marker is associated with a function to be executed with respect to the medical image data set and/or the image analysis result corresponding to the at least one visual marker. Further, the method further comprises the steps of generating a representation for display to a user on the basis of the modified image data, wherein the representation includes the at least one visual marker, displaying the representation in a graphical user interface, receiving a user input via the graphical user interface directed to the activation of the function, in response to receiving the user input, executing the function, generating a visualization of the result of the execution of the function, and displaying the visualization in the graphical user interface.

The representation may, in general be based on the image data of the medical image data set. In addition, the representation comprises the modified image data. For instance, the modified image data may be displayed as an overlay over the image data of the medical imaged data set. In particular, the representation may be a rendering on the basis of the medical image date and/or the modified image data. The visual markers may be of the same kind as described before. The associations between visual markers and functions may, for instance, be embedded in the processed medical image data set for later use.

The function may generally be configured to process medical image data and or image analysis results and generate a corresponding output on that basis.

By associating the visual markers with functions, the user is interactively offered ways to further process the medical image data and, thereby, come to a medical diagnosis. With that, a user can more easily and efficiently review the medical image data set for contents which are potentially relevant for making a medical diagnosis.

According to an aspect, the function is selected from a navigation function enabling to jump to a given position in the medical image data set, in particular, a position of the image analysis result, an image analysis function configured to automatically analyze image data associated with the at least one visual marker, an image enhancement function configured to enhance image data associated with the at least one visual marker, a content retrieval function configured to retrieve additional information relevant for the image analysis result associated with the at least one visual marker, a content push function configured to push the image analysis result associated with the at least one visual marker to a result database and/or an electronic medical report.

In particular, the image analysis function may be configured to measure a quantity associated with the underlying image data, such as a size, structure or type of a lesion.

In particular, the image enhancement function may be configured to process the image data such that it is better accessible to a user. For instance, the image enhancement function may be configured to change any one of a color, brightness, sharpness, etc. of the medical image data. Further the image enhancement function may be configured to enlarge a certain part of the image data (zooming).

In particular, the content retrieval function may be configured to analyze the image data and/or image analysis result associated with a given visual marker and retrieve additional contents (information) on that basis. For instance, this additional information may comprise information about similar patients, e.g., being associated with similar image data as the image data associated with the given visual marker. According to further examples, the additional information may comprise information about other medical image data sets of the patient that were acquired at a different point in time as the medical image data set. For instance, the additional information may comprise comparative image data from these other medical image data sets, the comparative image data depicting essentially the same region of the patient as the image data and/or image analysis result associated with the given visual marker. According to other examples, the additional information may comprise further diagnostic information such as a previous diagnosis associated with the image data and/or image analysis result associated with a given visual marker.

In particular, the content push function may be configured to transmit a certain information corresponding to a given visual marker to another medium for further processing in the diagnostic workflow. With that, the image analysis results may automatically be included in a medical report or archived in an appropriate data structure or database such as in a PACS.

With these functions, the user is provided with tools with which she or he can effectively gather additional information for coming to a medical diagnosis based on the medical image data set.

According to an aspect, the image analysis result relates to one or more medical findings depicted in the medical image data set. The method further comprises determining, for each medical finding, on or more characteristics, the characteristics being preferably selected from the group of: size, number per slice, severity, priority, malignancy, clinical significance, acuity, and/or progression of the medical finding. In the step of modifying, the image data is modified such that the one or more characteristics of the respective finding are encoded in the modified image data, preferably in connection with the included position indications.

The characteristics may be determined based on the image analysis results. For instance, if the image analysis results are included in the medical image data set, and object and/or character recognition may be employed as described before. Further, the image data associated with the image analysis result may be analyzed for one or more characteristics (if not already comprised in the image analysis result).

In particular, the one or more characteristics may be encoded in the aforementioned visual markers. For instance, the visual markers may comprise (or consist of) text describing the one or more characteristics. Further, the visual markers may comprise (or consist of) one or more pictograms indicating the one or more characteristics.

By visually encoding one or more characteristics of the image analysis result together with their position in the modified image data, the user is not only provided with an indication where important portions of the medical image data set are but also what content they have. Accordingly, a user may immediately jump to those portions of the medical image data set that are, according to the characteristics, most important. With that, a user can more easily and efficiently review the medical image data set for contents which are potentially relevant for making a medical diagnosis.

According to an aspect, the image analysis result is generated by applying at least one image analysis algorithm to the medical image data set and/or the image analysis result is retrieved form a database.

Accordingly, image analysis results either coming form a database or an image analysis algorithm can be incorporated. With that, existing results can effectively be considered and brought to the attention of the user by accordingly modifying the medical image data. With that, a user can more easily and efficiently review the medical image data set for contents which are potentially relevant for making a medical diagnosis.

According to an aspect, a system for modifying image data of a medical image data set is provided. The system comprises an interface unit and a computing unit. The interface unit is configured to obtain the medical image data set. The medical image data set depicts an image volume and comprises a plurality of slices with image data depicting a certain section of the image volume. The computing unit is configured to obtain at least one image analysis result applicable to the medical image data set (via the interface unit or by processing the medical image data set). The computing unit is further configured to determine a positional information of the image analysis result in the medical image data set, to modify the image data of one or more slices of the medical image data based on the positional information so that the modified image data depicts/indicates/highlights the position of the image analysis results in the medical image data set, and to provide the modified image data via the interface.

The computing unit may comprise a position determination unit configured to determine one or more positions or locations of the image analysis results in the medical image data set. To this end, the position determination unit may be configured to host, run and/or apply a corresponding image processing algorithm configured to recognize annotations in a medical image data set and provide positional information of the recognized annotations. The computing unit may comprise an image modification unit configured to modify medical image data based on the positional information. Optionally, the computing unit may comprise an image analysis unit configured to generate image analysis results based on medical image data sets (by processing medical image data sets). To this end, the image analysis unit may be configured to host, run and/or apply one or more corresponding image analysis algorithms. Optionally, the computing unit may further comprise a visualization unit configured to generate a visualization (for a user) based on the modified medical image data and the further medical data of the medical image data set.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories, and combinations thereof. The one or more memories may store instructions for carrying out the method steps according to the invention. The hardware can be configurable by the software and/or be operable by the software. Generally, all units, sub-units or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medical image data set. The interface unit may be further adapted to interface with one or more users of the system, e.g., by displaying the result of the processing by the computing unit to the user (e.g., in a graphical user interface) or by allowing the user to navigate through the medical image data set and/or interact with the information presented, for instance, by selecting one or more functions.

According to other aspects, the invention further relates to a processing system comprising the above system and a medical image system (or medical information system) configured to acquire, store and/or forward at medical image data sets. Thereby, the interface unit is configured to receive the medical image data set form the medical image system.

According to some examples, the medical image system comprises one or more archive stations for storing medical image data sets which may be realized as a cloud storage or as a local or spread storage, e.g., as a PACS (Picture Archiving and Communication System). Further, the medical image system may comprise one or more medical imaging modalities, such as a computed tomography system, a magnetic resonance system, an angiography (or C-arm X-ray) system, a positron-emission tomography system, a mammography system, or the like.

Optionally or additionally, the processing system may further comprise an image analysis system configured to generate one or more image analysis results based on medical image data sets. In suchlike embodiments, the image analysis would be conducted by a separate entity (separate in the sense that it is separate from the above computing unit). The image analysis system may be configured to host, run and/or apply one or more corresponding image analysis algorithms. Further, the image analysis system may be configured to receive a (original) medical image data set from the medical image system, process the original medical image data set so as to determine an image analysis result and include the image analysis result in the original medical image data set so as to generate the medical image data set, and provide the medical image data set to the system for modifying medical image data.

The systems may be adapted to implement the inventive method in their various aspects for modifying medical image data. The advantages described in connection with the method aspects may also be realized by the correspondingly configured systems' components.

According to another aspect, the present invention is directed to a computer program product comprising program elements which induce a computing unit of a system for modifying image data of a medical image data set to perform the steps according to one or more of the above method aspects, when the program elements are loaded into a memory of the computing unit.

According to another aspect, the present invention is directed to a computer-readable medium on which program elements are stored that are readable and executable by a computing unit of a system for modifying image data of a medical image data set according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts an embodiment of a system for modifying image data of a medical image data set;
FIG. 2 schematically depicts a method for modifying image data of a medical image data set according to an embodiment;
FIG. 3 schematically depicts an exemplary data flow diagram in connection with a method for modifying image data of a medical image data set according to an embodiment;
Fig. 4 schematically depicts a representation of the medical image data set including modified image data;
FIG. 5 schematically depicts method steps for modifying image data of a medical image data set according to an embodiment; and
Fig. 6 schematically depicts a method for modifying image data of a medical image data set according to a further embodiment.

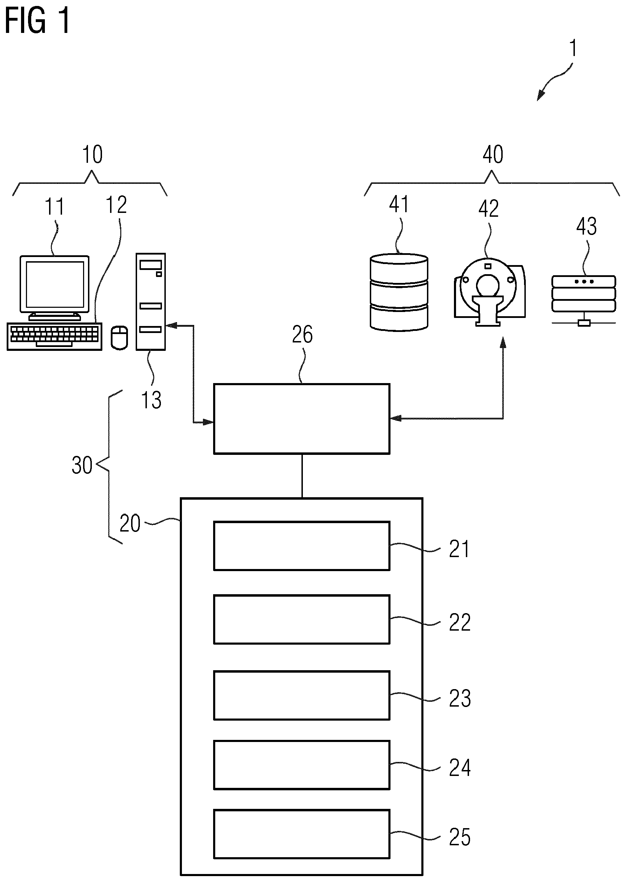

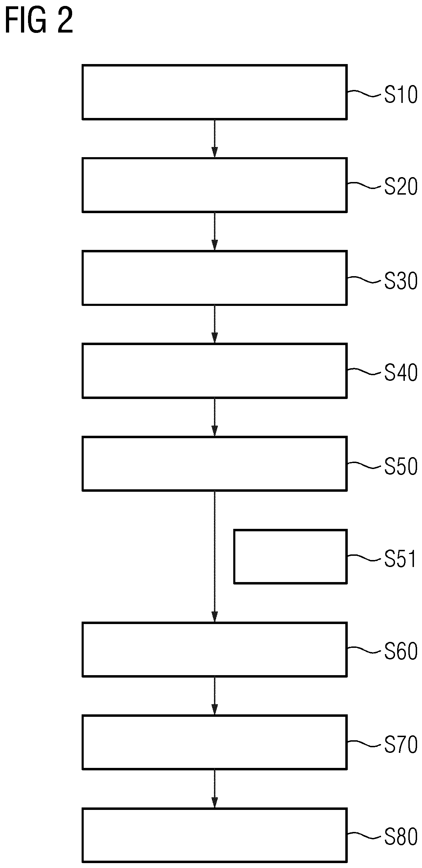

Figure 1 depicts a system 1 for modifying image data ID of a medical image data set MIDS. In this regard, system 1 is adapted to perform the methods according to one or more embodiments, e.g., as further described with reference to Figures 2 to 6. A user of system 1, according to some examples, may generally relate to a healthcare professional such as a physician, clinician, technician, radiologist, pathologist and so forth.

System 1 comprises a user interface 10 (as part of the interface unit) and a processing system 20 (as part of the computing unit 30). Further, system 1 may comprise or be connected to a medical information system 40. The medical information system 40 may generally be configured for acquiring and/or storing and/or forwarding and/or processing medical image data set MIDS.

For instance, medical information system 40 may comprise one or more archive/review stations 41 for storing medical image data sets MIDS. The archive/review stations 41 may be embodied by one or more databases. In particular, the archive/review stations 41 may be realized in the form of one or more cloud storage modules. Alternatively, the archive/review stations 41 may be realized as a local or spread storage, e.g., as a PACS (Picture Archiving and Communication System). According to some examples, medical information system 40 may also comprise one or more medical imaging modalities 42, such as a computed tomography system, a magnetic resonance system, an angiography (or C-arm X-ray) system, a positron-emission tomography system, an X-ray system, or the like. According to some examples, the medical information system 40 may comprise one or more image analysis systems 43 configured to generate one or more image analysis results IAR. To this end, the image analysis systems may apply one or more image analysis algorithms IAA to medical image data sets MIDS. The image analysis results IAR may designate the ensemble of the determined findings in a medical image data set MIDS. Thus, image analysis results IAR may generally comprise a plurality of different individual findings.

Medical image data sets MIDS may be three-dimensional image data sets acquired, for instance, using an X-ray system, a computed tomography system or a magnetic resonance imaging system or other systems. The image information may be encoded in a three-dimensional array of m times n times p voxels. Medical image data sets MIDS may include a plurality of image slices SL which are stacked in a stacking direction to span the image volume covered by the respective medical image data set MIDS.

An ensemble of voxels or pixels may be designated as image data ID of the respective medical image data set MIDS in the following. In general, any kind of imaging modalities and scanners may be used for acquiring such image data ID. Generally, a medical image data set MIDS may show a body part or an anatomical region or an anatomic object of a patient which may comprise various anatomies and organs. Considering the chest area as a body part, a medical image data set MIDS may, for instance, depict the lung lobes, the rib cage, the heart, lymph nodes, and so forth.

Medical image data sets MIDS may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as patient's name, ID, etc., and also special attributes containing the image pixel data ID and metadata, such as image analysis results IAR, extracted from the image data.

User interface 10 comprises a display unit 11 and an input unit 12. User interface 10 may be embodied by a mobile device such as a smartphone or tablet computer. Further, user interface 10 may be embodied as a workstation in the form of a desktop PC or laptop. Input unit 12 may be integrated in display unit 11, e.g., in the form of a touch screen. As an alternative or in addition to that, input unit 12 may comprise a keyboard, a mouse or a digital pen and any combination thereof. Display unit 11 may be configured for displaying the medical image data set MIDS and any results and images derived therefrom in the course of the method execution such as the modified image data m-ID and or the image analysis results IAR.

User interface 10 further comprises an interface computing unit 13 configured to execute at least one software component for serving display unit 11 and input unit 12 in order to provide a graphical user interface for allowing the user to select a target patient's case to be reviewed. In addition, interface computing unit 13 may be configured to communicate with medical information system 40 or processing system 20 for receiving a medical image data set MIDS and/or an image analysis result IAR related thereto. The user may activate the software component via user interface 10 and may acquire the software component, e.g., by downloading it from an internet application store. According to an example, the software component may also be a client-server computer program in the form of a web application running in a web browser. The interface computing unit 13 may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. User interface 10 may also be embodied as a client.

Processing unit 20 may comprise sub-units 21-25 configured to process the medical image data set MIDS and/or the image analysis result IAR, in order to partly modify the image data of the medical image data set MIDS so as to make the image analysis result IAR more accessible for a user upon reviewing the medical image data set MIDS.

Processing unit 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be a single device or multiple devices operating in serial, parallel, or separately. The processor may be a main processor of a computer, such as a laptop or desktop computer, or may be a processor for handling some tasks in a larger system, such as in the medical information system 40 or the server. The processor is configured by instructions, design, hardware, and/or software to perform the steps discussed herein. Alternatively, processing system 20 may comprise a real or virtual group of computers like a so called 'cluster' or 'cloud'. Such server system may be a central server, e.g., a cloud server, or a local server, e.g., located on a hospital or radiology site. Further, processing unit 20 may comprise a memory such as a RAM for temporally loading the medical image data set MIDS. Alternatively, such memory may as well be comprised in user interface 10.

Sub-unit 21 is a data retrieval module or unit. It is configured to access and search the medical information system 40 for the medical image data set MIDS and, optionally, any image analysis results IAR related to the medical image data set MIDS. Specifically, sub-unit 21 may be configured to formulate search queries and parse them to medical information system 40.

Sub-unit 22 can be conceived as a position determination module or unit. It is configured to obtain already existing image analysis results IAR associated with a medical image data set MIDS. It is further configured to process the image analysis results IAR so as to determine a position or location of one or more individual medical findings of the image analysis result IAR in the medical image data set MIDS. If the image analysis result IAR of a medical image data set MIDS is available separately from the medical image data set MIDS, sub-unit 22 may be configured to query the medical information system 40 accordingly. As an alternative, sub-unit 22 may activate sub-unit 25 to process the medical image data set MIDS so as to derive the image analysis result IAR therefrom. If the image analysis result IAR is included in the medical image data set MIDS, e.g., in the image data of the medical image data set MIDS, sub-unit 22 may be configured to recognize the image analysis result IAR in the medical image data set MIDS.

In general, sub-unit 22 may be configured to a apply a position-obtaining algorithm POA to the medical image data set MIDS, which position-obtaining algorithm is configured to determine a position of each finding of the image analysis result IAR in the medical image data set MIDS and provide these positions as positional information PI. Specifically, sub-unit 22 may be configured to apply a recognition algorithm as part of the position-obtaining algorithm to the medical image data set MIDS. For instance, the algorithm may be an object and/or character recognition algorithm such as an OCR algorithm. After having obtained the image analysis result IAR, sub-unit 22 may be configured to locate the different parts of the image analysis result IAR in the medical image data set MIDS. In other words, sub-unit 22 may be configured to derive positional information indicating the position of the individual parts of the image analysis result IAR in the medical image data set MIDS. The position may be a slice position and/or a location within a respective slice SL.

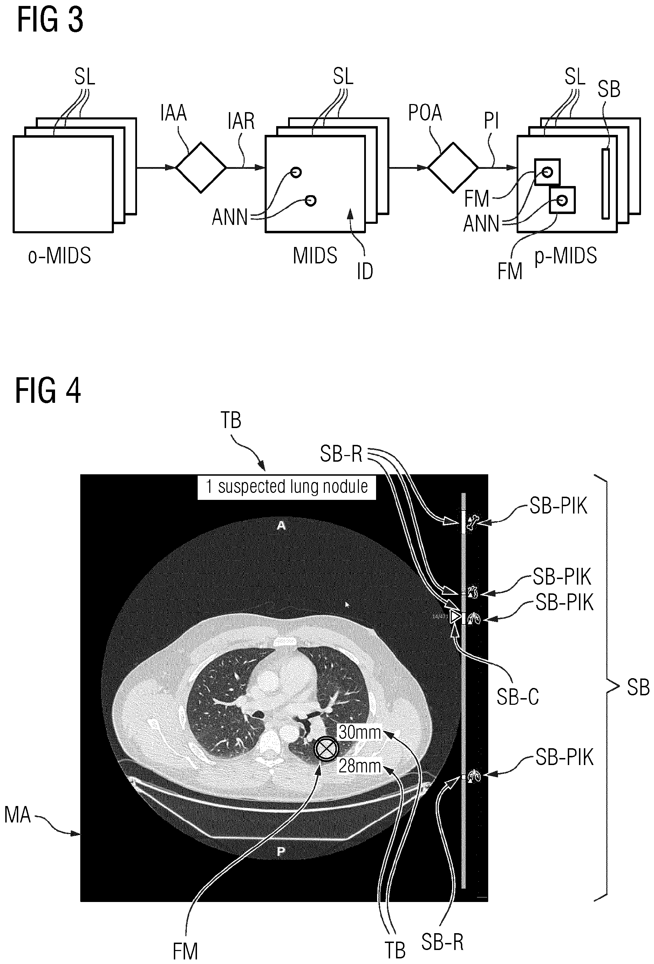

Sub-unit 23 may be conceived as a image modification module or unit. Sub-unit 23 may configured to take image data ID comprised in the medical image data set MIDS and modify it, based on the positional information PI, to generate modified image data m-ID in order to highlight the image analysis result IAR for a user. To do so, sub-unit 23 may be configured to add visual clues SB, MA, TB, FM into the medical image data set MIDS to make it easier for a user to find relevant image analysis results IAR when scrolling through the medical image data set MIDS. Examples for suchlike clues SB, MA, TB, FM are shown in Figure 4.

Sub-unit 24 is a visualization module configured to translate or convert the modified image data m-ID into a suitable representation for displaying to the user. The suitable representation can be in the form of a rendering in which the image analysis result IAR is visually highlighted. This may mean that the image analysis result IAR is enhanced in the visualization. Moreover, sub-unit 24 may be configured to highlight the image analysis result IAR in the form of symbols or labels SB, MA, TB, FM in the medical image data set MIDS.

Sub-unit 25 is an image analysis module or unit. Sub-unit 25 is configured to analyze medical image data sets MIDS to derive image analysis results IAR thereform. To this end, sub-unit 25 may be configured to run/host/apply one or more image analysis algorithms IAA to medical image data sets MIDS. For instance, suchlike image analysis algorithms IAA may be configured to recognize and/or classify lesions in medical image data. To provide an example, the image analysis algorithms IAA may comprise lung CAD algorithms configured to extract lung-lesions from medical image data ID depicting the lung of a patient. Sub-unit 25 is optional in that the image analysis result-generation may also happen outside the processing unit 20, for instance, in the medical information system 40.

The designation of the distinct sub-units 21-25 is to be construed by way of example and not as limitation. Accordingly, sub-units 21-25 may be integrated to form one single unit (e.g., in the form of "the computing unit 30") or can be embodied by computer code segments configured to execute the corresponding method steps running on a processor or the like of processing system 20. The same holds true with respect to interface computing unit 13. Each sub-unit 21-25 and interface computing unit 13 may be individually connected to other sub-units and/or other components of the system 1 where data exchange is needed to perform the method steps. For example, sub-units 21 and 25 may be connected via a data interface 26 to medical information system 40. Likewise, data interface 26 may connect the sub-units 21 to 25 to interface computing unit 13 for forwarding the results of the computation to the user and collect user inputs.

Processing system 20 and interface computing unit 13 together may constitute the computing unit 30. Of note, the layout of computing unit 30, i.e., the physical distribution of interface computing unit 13 and sub-units 21-25 is, in principle, arbitrary. For instance, sub-unit 24 (or individual elements of it or specific algorithm sequences) may likewise be localized in user interface 10. The same holds true for the other sub-units 21-25. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as "frontend" or "client" facing the user, while processing system 20 could then be conceived as "backend" or server. Communication between user interface 10 and processing system 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via data interface 26. For example, processing system 20 may be activated on a request-base, wherein the request is sent by user interface 10. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Data interface 26 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Data interface 26 for data exchange together with the components for interfacing with the user 11, 12 may be regarded as constituting an interface unit of system 1.

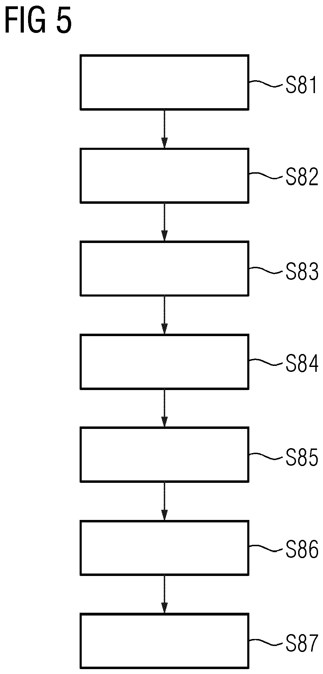

Figure 2 depicts a method for modifying image data ID of a medical image data set MIDS according to an embodiment. Corresponding data streams are illustrated in Figure 3. Additional optional sub-steps according to some embodiments are shown in Figure 5. The method comprises several steps. The order of the steps does not necessarily correspond to the numbering of the steps but may also vary between different embodiments of the present invention. Further, individual steps or a sequence of steps may be repeated.

In Figure 2 it is assumed that the image analysis result IAR is included in the medical image data set MIDS. The image analysis result IAR has been generated based on an original medical image data set o-MIDS. Specifically, the original medical image data set o-MIDS is processed and the ensuing image analysis result IAR has been inserted in the image data ID of the original medical image data set o-MIDS. The original medical image data set o-MIDS with thus adapted image data ID is the medical image data set MIDS which is to be enhanced by highlighting the image analysis result IAR in the course of the further image data modification according to the invention.

In a first step S10, the original medical image data set o-MIDS is obtained. The original medical image data set o-MIDS can be seen as the target image series which is to be analyzed in the diagnostic workflow. This may involve selecting the original medical image data set o-MIDS from a plurality of cases, e.g., stored in the medical information system 40. The selection may be performed manually by a user, e.g., by selecting appropriate files in a graphical user interface running in the user interface 10.

At step S20, an image analysis results IAR is generated. This may involve providing an image analysis algorithm IAA configured to analyze the original medical image data set o-MIDS and provide a corresponding image analysis results IAR. Considering as an example that the original medical image data set o-MIDS displays the chest area of a patient, the image analysis algorithm IAA may be configured to automatically detect lung lesions.

At step S30, the image analysis result IAR is included in the original medical image data set o-MIDS. This may mean including one or more annotations ANN indicating the image analysis result IAR in the image data ID of the original medical image data set o-MIDS. In other words, the image analysis result IAR is "burnt-in" into the original medical image data set o-MIDS. Of note, this is the standard procedure followed by many commercially available image analysis algorithms IAA. Specifically, the medical image data set MIDS may comprise one or more DICOM secondary captured images. The thus processed original medical image data set o-MIDS is the medical image data set MIDS to be further processed in the subsequent steps.

Steps S10, S20, and S30 are optional and not essential to the following method steps. This is because the medical image data set MIDS may be provided completely independently from the following processing of the medical image data set MIDS. In particular, the medical image data set MIDS may be provided by entirely different systems well in advance of the further processing of steps S40 ff.

At step S40, the medical image data set MIDS is obtained. As explained in connection with step S10, this may involve selecting the medical image data set MIDS from a plurality of (already processed) cases, e.g., stored in the medical information system 40. The selection may be performed manually by a user, e.g., by selecting appropriate files in a graphical user interface running in the user interface 10. Alternatively, the medical image data set MIDS may be obtained directly from the image analysis algorithm IAA.

At step S50, the image analysis result IAR is obtained from the medical image data set MIDS. As the image analysis result IAR is included in the image data of the medical image data set MIDS, step S50 involves processing the image data ID of the medical image data set MIDS so as to recognize the image analysis result IAR (or the annotations ANN included in the medical image data set MIDS). To this end, object and/or character recognition algorithms may be used. These algorithms may be configured to recognize characters and objects in image data, e.g., by analyzing pixel values of the individual slices comprised in the medical image data set MIDS. For instance, OCR algorithms may be used for that purpose. Optionally, step S50 may involve determining one or more super-ordinate characteristics of the findings associated with the annotations ANN comprised in the medical image data set MIDS at step S51. These characteristics may comprise the type and/or property of individual findings comprised in the medical image data set MIDS. Here, the information as extracted by the as well as underlying image data of the medical image data set MIDS may be evaluated.

At step S60, a positional information PI of the image analysis result IAR is determined. For each annotations ANN, this may comprise determining a slice position and a location within the respective slice SL. Once the image analysis result IAR is obtained, the determination of the positional information PI is straight forward. According to some examples, the positional information PI may be obtained as part of the object recognition at step S50. The positional information PI may be determined by a correspondingly adapted position-obtaining algorithm POA. The position-obtaining algorithm POA may generally be configured to intake a medical image data set MIDS and/or an image analysis result IAR and output the corresponding positional information PI. Optionally, the position-obtaining algorithm POA may comprise an object and/or character recognition algorithm.

At step S70, the positional information PI is used to modify the image data ID of the medical image data set MIDS to make the image analysis result IAR more accessible for a user. Some examples of such a modification are shown in Figure 4. In general, the processing in step S70 may result in some of the image data of the medical image data set MIDS being modified to form modified image data p-ID.

At step S80, the modified image m-ID data is provided. For instance, the modified image m-ID data may be included in the medical image data set MIDS so as to from a processed medical image data set p-MIDS with the processing result "burnt-in" into the processed medical image data set p-MIDS. As an alternative or in addition to that, the modified image data MID may be used for rendering a representation of the medical image data set MIDS for displaying to the user via the graphical user interface. Since the representation comprises the modified image data m-ID, the user is provided with an indication about the position of the image analysis result IAR in the medical image data set MIDS.

In Figure 4, an exemplary representation of the processed medical image data set p-MIDS with visual clues for the user regarding the position of the image analysis result IAR is shown. For illustration purposes, Figure 4 includes various different ways how the image data ID of the medical image data set MIDS may be modified, e.g., with visual markers, pictograms, highlights, or scrollbars. Self-speaking these elements may also be used separately or in arbitrary combinations.

On the right side, a burnt-in scrollbar SB is depicted which is used as a navigation indicator for the presence of image analysis results IAR. The scrollbar SB may include various visual markers SB-C, SB-R, SB-PIK. For instance, one of these visual markers SB-C may indicate the position of the slice SL currently looked at in the medical image data set MIDS. Other visual markers SB-R may indicate slices SL or regions of slices SL, where an image analysis result IAR and/or annotations ANN and/or findings are present. Further, the visual markers may include symbols or pictograms SB-PIK indicating one or more characteristics of the respective findings. For instance, the visual markers SB-PIK may correspond to, for example:
- the amount/number of detected findings in a slice SL or region of slices SL,
- the spatial extent of detected findings (one slice SL vs. several neighboring slices SL),
- the type of findings or the concerned organ or anatomical structure (lesion, pneumothorax, PE, heart/aorta, pathology finding, etc.),
- the criticality or priority of the findings (pneumothorax, brain hemorrhage, infarct, aortic dilatation, etc.)

It is envisioned that the scrollbar SB is included in all of the slices SL of the medical image data set MIDS. In each instance of the scrollbar SB throughout the medical image data set MIDS, only the visual marking of the slice currently looked at is altered. Optionally, several burnt-in scroll bars, e.g., one per organ: lungs, heart, etc., may be included in the medical image data set MIDS by way of the modified image data m-ID.

As another way to visually highlight the image analysis result IAR and/or annotations ANN and/or findings the findings, a styled border or margin MA may be used to visibly identify slices SL which contain findings, and to differentiate them instantaneously from slices SL which do not contain findings. For instance, margins having a distinct color, thickness, line-style, shading, etc. may be used.

Additionally or alternatively, visual markers may be text boxes TB, relating to the visualized slice SL, succinctly describing which finding types/locations/etc. have been detected in the image analysis result IAR. Further, a set of finding markers FM, measurements, etc. that are burnt-in in the image pixel data relating to the findings contained in the image analysis result IAR may be used to visualize or highlight in detail the finding(s) in question that are visible on a particular slice SL. Here, the same symbols or pictograms as described before may be used.

Optionally, sets of findings may be grouped or boxed together (e.g., for chest imaging: parenchymal findings, pleural findings, cardiac, bones, soft tissue, etc.). Optionally, a clinical significance or acuity can be encoded (e.g., through different colors or font sizes). Optionally, a spatial extent of a finding could be encoded (e.g., by way of font sizes, different markers, etc.).

While Figure 4 concerns the static use of the positional information to include burnt-in markers in the image data ID of the medical image data set MIDS, it is also possible to use the information in an interactive manner.

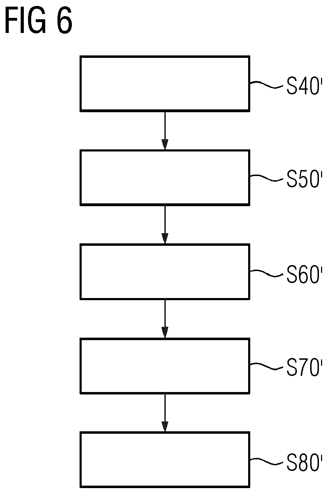

A corresponding method according to an embodiment is shown in Figure 5. The steps of Figure 5 may be incorporated in the super-ordinate methods schematically depicted in Figures 2 or 6. For instance, the steps of Figure 5 may follow the provision of the modified image data m-ID at step S80. The method of Figure 5 comprises several steps. The order of the steps does not necessarily correspond to the numbering of the steps but may also vary between different embodiments. Further, individual steps or a sequence of steps may be repeated.

At step S81, at least one visual marker SB, SB-PIK, SB-C, SB-R, MA, FM, TB of the modified image data is associated with a processing function. The processing function may be an image processing or a data processing function. For instance, the function may be an image analysis function configured to measure a quantity associated with the underlying image data, such as a size, structure or type of a lesion. According to other examples, the function may be an image enhancement function configured to process the image data such that it is better accessible to a user and may, for instance, include a zoom function. According to other examples, the function may be a content retrieval function configured to analyze the image data and/or image analysis result associated with a given visual marker and retrieve additional contents (information) on that basis. According to other examples, the function may be a content push function configured to transmit a certain information corresponding to a given visual marker to another medium for further processing in the diagnostic workflow. According to other examples, the function may be a navigation function enabling to jump to a given position in the medical image data set MIDS, p-MIDS upon clicking on it.

At step S82, a representation for displaying to a user is generated. The representation includes the visual markers that are associated with the above functions. Accordingly, an interactive display of data is effected which helps the user to more efficiently review the medical image data set MIDS, p-MIDS.

For instance, an interactive scrollbar SB may be provided which may enable the following functionalities:
- jumping to the corresponding slice SL (and/or zoom onto the finding) when clicking on a findings symbol SB-R, SB-PIK in the interactive scrollbar SB, and/or
- obtaining more detailed information on the finding by hovering over a finding symbol SB-R, SB-PIK in the interactive scrollbar (e.g., with a mouse).

Further, interactive markers may be added, which enable superimposing finding details, e.g., shown as an overlay on the image data ID, m-ID, e.g., by clicking on the marker. Similarly, zooming functions may be added that enable enlarging a certain region of the image data ID, m-ID by activating the underlying function. Optionally, an editing window may be provided, which may enable editing attributes attached to the finding. Optionally, upon clicking, the finding could be presented as a thumbnail with appropriate image processing steps (reformatting, MIPs, windowing, contour insertion, etc.). Optionally, upon clicking, the interactive markers could also enable triggering added diagnostic tools (e.g., malignancy scoring of a pulmonary nodules). Optionally, upon clicking, the interactive markers could also enable a notification through phone or email to the clinician and alert them of an acute/emergent finding or bring to their attention a clinically significant finding that requires further follow up. Optionally, findings can be actively rejected/removed (if deemed a false positive by the user). Optionally, upon clicking, findings can be sent to a reporting system. Optionally, upon clicking, findings could display the same anatomic location of a previous scan of the same patient or another scan of the same patient (acquired with the same or a different modality).

At step S83, the representation generated at step S84 may be displayed and, thus, provided to a user. For instance, the displaying may happen through a graphical user interface running on the user interface 10.

That followed, at step S84, a user input directed to activate one or more functions associated with a certain visual marker may be received. The user input may be a mouse click, a hovering over a visual marker with an input device, a gesture command, a voice command, and/or a keyboard or touch pad command, and the like.

At step S85, the function the user sought to activate with the user input is executed and the corresponding processing result is obtained. That followed, a visualization is generated based on this processing result in step S86 and displayed to the user in step S87. Depending on the function, this may be an enlarged view, supplementary image data, additional descriptions/captions etc.

Figure 6 depicts a method for modifying image data of a medical image data set MIDS according to a further embodiment. Additional optional sub-steps according to some embodiments are shown in Figure 5. The method comprises several steps. The order of the steps does not necessarily correspond to the numbering of the steps but may also vary between different embodiments. Further, individual steps or a sequence of steps may be repeated.

In contrast to the embodiment shown in Figures 2 and 3 it is assumed in Figure 6 that the image analysis result IAR is available separately from the medical image data set MIDS. For instance, the image analysis result IAR may directly be provided by an image analysis algorithm IAA or retrieved from a database of the medical information system 40. In other words, the medical image data set MIDS does not comprise annotations ANN relating to the image analysis result IAR.

At step S40', the medical image data set MIDS is obtained. Step S40' corresponds to step S40 of Figure 2 - with the exception that the medical image data set MIDS obtained at step S40' does not comprise annotations ANN relating to the image analysis result IAR.

At step S50', an image analysis result IAR relating to the medical image data set MIDS is obtained. For instance, one or more databases of the medical information system 40 may queried for applicable image analysis results IAR. The image analysis result IAR may have been independently derived from the medical image data set MIDS, e.g., by applying one or more image analysis algorithms IAA as described before. Moreover, the image analysis result IAR may have been obtained from medical image data different from the medical image data set MIDS, for instance, from image data of different (earlier) examinations of the patient or image data of similar patients having a similar clinical indication as the patient of the medical image data set MIDS.

At step S60', the positional information PI is determined. As the image analysis result IAR is not inscribed in the medical image data set MIDS, it can be directly extracted from the image analysis result IAR. For instance, this information may be provided as metadata associated to the image analysis result IAR or provided directly by the image analysis algorithm IAA.

At step S70' the image data ID of the medical image data set MIDS is modified essentially as described in connection with Figure 2.

At step S80', the modified image data m-ID is provided essentially as described in connection with Figure 2. Further, also the interactive visualization explained in connection with Figure 5 may ensue.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

## Claims

1. Computer-implemented method for modifying image data (ID) of a medical image data set (MIDS) with the following steps:
- obtaining (S40, S40') the medical image data set (MIDS), the medical image data set (MIDS) depicting an image volume and comprising a plurality of slices (SL) with image data (ID) depicting a certain section of the image volume,
- obtaining (S50, S50') at least one image analysis result (IRA) applicable to the medical image data set (MIDS),
- determining (S60, S60') a positional information (PI) of the image analysis result (IAR) in the medical image data set (MIDS),
- modifying (S70, S70') image data (ID) of one or more slices (SL) of the medical image data set (MIDS) based on the positional information (PI) so that the modified image data (m-ID) indicates the position of the image analysis results (IAR) in the medical image data set (MIDS),
- providing (S80, S80') the modified image data (m-ID).

2. Method according to claim 1, wherein
- the step of providing (S80, S80') the modified image data (m-ID) comprises generating a processed medical image data set (p-MIDS) based on the medical image data set (MIDS) and the modified image data (m-ID) and providing the processed medical image data set (p-MIDS).

3. Method according to any one of the preceding claims, wherein
- the at least one image analysis result (IAR) is included in the medical image data set (MIDS) in the form of one or more annotations (ANN) on one or more slices (SL) of the medical image data set (MIDS).

4. Method according to any one of the preceding claims, wherein
the image data (ID) of the plurality of slices (SL) respectively comprises a plurality of pixels each having a pixel value, and
the step of modifying (S70, S70') comprises changing one or more pixel values of the one or more slices (SL).

5. Method according to any one of the preceding claims, wherein
in the step of modifying (S70, S70'), in each of the slices (SL) comprised in the medical image data set (MIDS) image data (ID) is modified.

6. Method according to any one of the preceding claims, wherein
- the step of modifying (S70, S70') comprises modifying image data (ID) of the slices (SL) of the medical image data set (MIDS) so that, in each slice (SL), the modified image data (m-ID) depicts the current slice position (SB-C) of the respective slice in the medical image data set (MIDS) and, relative thereto, the slice position (SB-R) of the image analysis results (IAR).

7. Method according to claim 6, wherein
- the step of modifying (S70 S70') comprises modifying the image data (ID) of the slices (SL) such that each slice (SL) depicts a scrollbar (SB) representing the slices (SL) of the medical image data set (MIDS), and
- the slice position (SB-C) of the respective slice (SL) and the slice position (SB-R) of the image analysis result (IAR) are respectively encoded in the scrollbar (SB) by visual markers (SB-C, SB-R).

8. Method according to any one of the preceding claims, wherein
the step of modifying (S70, S70') comprises inserting one or more visual markers (SB-C, SB-R, SB-PIK, MA, TB, FM) in the image data (ID) of the medical image data set (MIDS).

9. Method according to any one of claims 7 or 8, wherein
- at least one visual marker (SB-C, SB-R, SB-PIK, MA, TB, FM) is associated with a function to be executed with respect to the medical image data set (MIDS) and/or the analysis result (IRA) corresponding to the at least one visual marker (SB-C, SB-R, SB-PIK, MA, TB, FM), and
- the method further comprises the steps of
- generating (S82) a representation for display to a user on the basis of the modified image data (m-ID) the representation including the at least one visual marker (SB-C, SB-R, SB-PIK, MA, TB, FM),
- displaying (S83) the representation in a graphical user interface,
- receiving (S84) a user input via the graphical user interface directed to the activation of the function,
- in response to receiving the user input executing (S85) the function,
- generating (S86) a visualization of the result of the execution of the function, and
- displaying (S87) the visualization in the graphical user interface.

10. Method according to claim 9, wherein the function is selected from:
- a navigation function enabling to jump to a given position in the medical image data set (MIDS), in particular, a position of the image analysis result (IAR),
- an image analysis function configured to automatically analyze image data (ID) associated with the at least one visual marker (SB-C, SB-R, SB-PIK, MA, TB, FM),
- an image enhancement function configured to enhance image data (ID) associated with the at least one visual marker (SB-C, SB-R, SB-PIK, MA, TB, FM),
- a content retrieval function configured to retrieve additional information relevant for the image analysis result (IAR) associated with the at least one visual marker (SB-C, SB-R, SB-PIK, MA, TB, FM),
- a content push function configured to push the image analysis result (IAR) associated with the at least one visual marker to a database (41) and/or an electronic medical report.

11. Method according to any one of the preceding claims, wherein
- the image analysis result (IAR) comprises one or more medical findings depicted in the medical image data set (MIDS),
- the method further comprises determining, for each medical finding, one or more characteristics, the characteristics being preferably selected from the group of: a size, severity, priority, malignancy, clinical significance, acuity, and/or progression of the medical finding, and/or the number of medical findings per slice, and
- in the step of modifying (S70, S70'), the image data (ID) is modified such that the one or more characteristics of the respective finding are encoded in the modified image data (m-ID) .

12. Method according to any one of the preceding claims, wherein
- the image analysis result (IAR) is generated by applying at least one image analysis algorithm (IAA) to the medical image data set (MIDS), and/or
- the image analysis result (IAR) is retrieved form a database (41).

13. System (1) for modifying image data (ID) of a medical image data set (MIDS), the system (1) comprising an interface unit (11, 12, 26) and a computing unit (30),
- the interface unit (11, 12, 26) being configured to:
- obtain the medical image data set (MIDS), the medical image data set (MIDS) depicting an image volume and comprising a plurality of slices (SL) each with image data (ID) depicting a certain section of the image volume,
- the computing unit (30) being configured to:
- obtain at least one image analysis result (IAR) applicable to the medical image data set (MIDS) via the interface unit (11, 12, 26) and/or by processing the medical image data set (MIDS),
- determine a positional information (PI) of the image analysis result (IAR) in the medical image data set,
- modify the image data (ID) of one or more slices (SL) of the medical image data set (MIDS) based on the positional information (PI) so that the modified image data (mID) indicates the position of the image analysis results (IAR) in the medical image data set (MIDS), and
- provide the modified image data (m-ID) via the interface unit (11, 12, 26).

14. Computer program product comprising program elements which induce a computing unit (30) of a system (1) for modifying image data (ID) of a medical image data set (MIDS) to perform the steps according to the method of any of claims 1 to 12, when the program elements are loaded into a memory of the computing unit (30).

15. Computer-readable medium on which program elements are stored that are readable and executable by a computing unit (30) of a system (1) for modifying image data(ID) of a medical image data set (MIDS) to perform steps of the method according to any of claims 1 to 12, when the program elements are executed by the computing unit (30).
